(19) Europäisches Patentamt

European Patent Office

Office européen des brevets

(11) **EP 1 171 079 B1**

(12) **FASCICULE DE BREVET EUROPEEN**

(45) Date de publication et mention
de la délivrance du brevet:
**02.03.2005 Bulletin 2005/09**

(51) Int Cl.[7]: **A61K 6/10**, C08K 9/04,
C08K 9/06

(21) Numéro de dépôt: **00917123.2**

(22) Date de dépôt: **05.04.2000**

(86) Numéro de dépôt international:
**PCT/FR2000/000853**

(87) Numéro de publication internationale:
**WO 2000/061074 (19.10.2000 Gazette 2000/42)**

(54) **MATERIAU ELASTOMERE SILICONE HYDROPHILE UTILISABLE NOTAMMENT POUR LA PRISE D'EMPREINTES DENTAIRES**

HYDROPHILE SILIKONELASTOMER-ZUSAMMENSETZUNG UND SEINE VERWENDUNG ALS ABFORMMATERIAL

HYDROPHILE SILICONE ELASTOMER MATERIAL USED IN PARTICULAR FOR IMPRESSIONS

(84) Etats contractants désignés:
**AT BE CH DE DK ES FI FR GB GR IE IT LI LU NL PT SE**

(30) Priorité: **09.04.1999 FR 9904675**

(43) Date de publication de la demande:
**16.01.2002 Bulletin 2002/03**

(73) Titulaire: **RHODIA CHIMIE**
**92408 Courbevoie Cédex (FR)**

(72) Inventeurs:
• **DEL TORTO, Marco**
**I-21058 Solbiate Olona (IT)**
• **HOWE, Fabienne**
**F-69300 Caluire (FR)**
• **POUCHELON, Alain**
**F-69330 Meyzieu (FR)**
• **PUSINERI, Christian**
**F-69360 Serezin du Rhone (FR)**

(74) Mandataire: **Trolliet, Maurice et al**
**RHODIA SERVICES**
**Direction de la Propriété Industrielle**
**CRIT-Carrières - BP 62**
**69192 Saint-Fons Cédex (FR)**

(56) Documents cités:
**EP-A- 0 480 238** **WO-A-96/32088**
**WO-A-98/58997**

## EP 1 171 079 B1

**Description**

Le domaine de la présente invention est celui des matériaux silicones comprenant une composition polyorganosiloxane (en abréviation POS) réticulable ou durcissable en élastomère silicone par des réactions de polyaddition ainsi qu'un agent mouillant permettant de conférer audit matériau un caractère hydrophile. Les applications visées par de tels systèmes sont, notamment, la prise d'empreintes et, plus particulièrement, la prise d'empreintes dentaires dans le cadre de la réalisation de prothèses. Par l'expression "prise d'empreintes", on entend définir dans le présent mémoire : non seulement les opérations de prise d'empreintes de n'importe quel objet et de n'importe quelle forme pour réaliser un modèle en particulier en plâtre ; mais encore les opérations de reproductions ou de duplications de modèles en particulier en plâtre. Par l'expression "prise d'empreintes dentaires", on entend définir dans le présent mémoire : non seulement les opérations où l'on procède à la prise d'empreintes dentaires en bouche pour obtenir des reproductions exactes de mâchoires ou de parties de mâchoires portant ou non, et en totalité ou en partie, des dents et pour former des modèles en plâtre ; mais encore les opérations de duplications où l'on procède à la reproduction de modèles de mâchoires ou de parties de mâchoires en plâtre dans un laboratoire de prothèse dentaire. Les applications visées englobent encore, en particulier, la fabrication de tampons tels que ceux utilisés dans les techniques de tampographie.

La présente invention a également pour objet un procédé de préparation du matériau élastomère silicone hydrophile. L'invention vise encore l'utilisation dudit matériau pour la prise d'empreintes, par exemple dentaires. Enfin, l'invention vise l'utilisation dudit matériau pour la fabrication de tampons tels que ceux utilisés dans les techniques de tampographie.

L'utilisation des matériaux silicones est largement répandue dans ces domaines. Ceci est dû en partie au fait que les matériaux silicones offrent, d'une part une grande diversité de caractéristiques chimiques, mécaniques et physiques, et, d'autre part, un caractère non toxique, non irritant et non allergisant. En outre, les matériaux silicones constituent de piètres substrats de culture pour les micro-organismes, ce qui leur confère des aptitudes remarquables au regard de l'hygiène.

Les compositions POS auxquelles on s'intéresse dans le cadre de la présente invention comprennent au minimum :

- un POS (1) porteur de fonctions Si-alcényles aptes à réagir par des réactions d'addition avec les fonctions réticulantes Si-H d'un POS (2),
- un POS (2) porteur de fonctions Si-H apte à réagir avec les fonctions Si-alcényles du POS (1),
- éventuellement un POS (3) non réactif, différent des POS (1) et (2), utilisable comme diluant,
- un catalyseur des réactions de polyaddition, et
- une charge minérale renforçante particulaire, généralement de nature siliceuse, traitée par un agent de compatibilisation à base notamment d'un organosilane ou d'un organosilazane, et éventuellement une charge semi-renforçante ou de bourrage.

On sait que de pareilles compositions POS, pouvant avantageusement se présenter sous forme de deux composants, sont réticulables ou durcissables à température ambiante et sont particulièrement intéressantes dans le domaine des prises d'empreintes, en particulier des prises d'empreintes dentaires car ces compositions sont douées de propriétés de fluidité et de filmogénéité avant réticulation, ce qui rend possible la prise d'une empreinte de n'importe quelle forme avec une excellente reproduction des détails. Par ailleurs, ces compositions peuvent réticuler par des réactions de polyaddition en quelques minutes à température ambiante ; de plus, elles sont non toxiques et satisfont aux réglementations européennes en matière pharmaceutique. La réticulation qui entraîne le durcissement de la composition silicone permet de constituer des moules en élastomères offrant des propriétés mécaniques, une stabilité dimensionnelle et une tenue thermique qui sont conformes aux spécifications souhaitées.

Toutefois, les compositions pour empreintes à base de silicone réticulant par des réactions de polyaddition sont intrinsèquement hydrophobes. Ainsi donc, lorsqu'on applique la masse de moulage mélangée à la surface humide des dents et des gencives, il peut y avoir un défaut de coulée ou bien une pénétration insuffisante dans les creux des gencives en raison de la présence de résidus de liquides ; après réticulation, la reproduction est donc défectueuse. D'autre part, lorsqu'au cours des opérations de duplication du positif de l'empreinte en plâtre on est amené à couler, dans un moule en silicone hydrophobe, du plâtre réfractaire à caractère hydrophile, il peut y avoir occlusion de petites bulles d'air en raison de l'incompatibilité entre les surfaces ; cette occlusion conduit à la réalisation d'une reproduction défectueuse.

On peut pratiquement éliminer ces inconvénients en conférant aux compositions POS intrinsèquement hydrophobes, un caractère hydrophile grâce à l'emploi de différents agents tensioactifs ; c'est ainsi que l'on propose d'utiliser : dans US-A-4.657.959 : un polyorganosiloxane à fonctions polyéthers ; dans US-A-4.691.039 et US-A-4.752.633 : un silane éthoxylé ; dans US-A-5.064.891 : un polyorganosiloxane à fonctions polyols ; dans EP-A-0.480.233 : un alcool gras poly(oxyalkylé) ; dans FR-A-2.600.886 : une protéine soluble dans l'eau associée éventuellement à un agent tensioactif non ionique.

Poursuivant des recherches dans ce domaine de la technique, la demanderesse a constaté :

- que l'introduction d'agent(s) tensioactif(s) dans une composition POS de polyaddition, renfermant une charge constituée, en tout ou partie, par une charge minérale renforçante traitée,
- conduit au développement d'un caractère thixotrope, c'est-à-dire à une augmentation significative de la viscosité du matériau silicone à l'état non réticulé comprenant comme éléments constitutifs la composition POS et le (ou les) agent(s) tensioactif(s) : le matériau silicone devient de ce fait insuffisamment coulant et cet inconvénient est de nature à gêner considérablement les opérations de prise d'empreintes et à dégrader la qualité de la reproduction des détails.

Ce phénomène de thixotropie, lié à l'introduction de tensioactif(s) dans les compositions POS à charges minérales renforçantes traitées, se manifeste de façon particulièrement néfaste pour les matériaux silicones dont la teneur en charge minérale renforçante traitée est importante, par exemple égale ou supérieure à environ 15 % par rapport au poids du matériau silicone. Cette borne d'environ 15 % peut être abaissée vers environ 10 % quand le matériau silicone renferme par ailleurs une charge semi-renforçante ou de bourrage à raison de 10 % à 30 % par rapport au poids du matériau silicone.

Il a maintenant été trouvé qu'il est possible de contourner cette difficulté en utilisant, pour préparer le matériau silicone, une charge minérale renforçante traitée, particulière, qui se présente sous la forme d'une suspension obtenue en réalisant un traitement en deux temps de la charge par un agent de compatibilisation (en abréviation AC), et en opérant ce traitement en présence d'au moins une portion du POS (1) porteur de fonctions Si-alcényles.

La conjonction de l'emploi de la charge minérale renforçante traitée de la manière particulière indiquée ci-avant et de remploi d'un ou plusieurs agent(s) tensioactif(s) conduit à un matériau silicone de polyaddition utilisable notamment pour prise d'empreintes offrant le compromis des caractéristiques recherchées : fluidité (à l'état non réticulé) et hydrophilie (à l'état non réticulé comme à l'état réticulé) et hautes propriétés mécaniques (à l'état réticulé).

D'où il s'ensuit que la présente invention, prise dans son premier objet, concerne un matériau silicone, utilisable notamment pour la prise d'empreintes, par exemple dentaires, qui comprend les constituants suivants :

**I.** une composition POS réticulable par des réactions de polyaddition comprenant :

(1) au moins un POS porteur de fonctions Si-alcényles aptes à réagir par des réactions d'addition avec les fonctions réticulantes Si-H d'un POS (2),

(2) au moins un POS porteur de fonctions Si-H aptes à réagir avec les fonctions Si-alcényles du POS (1),

(3) éventuellement au moins un POS non réactif, différent des POS(1) et (2), utilisable comme diluant;

(4) un catalyseur des réactions de polyaddition,

(5) une charge minérale renforçante particulaire traitée par un agent de compatibilisation (AC) ;

ladite composition ayant une viscosité dynamique inférieure à 10 000m Pa.S mesurée à l'aide d'un viscosimètre BROOKFIELD selon les indications de la norme AFNOR NFT 76106 de mai 1982.

**II.** un agent mouillant consistant dans un ou plusieurs agents(s) tensioactifs permettant de conférer un caractère hydrophile à la surface du matériau silicone ;

ledit matériau silicone étant caractérisé en ce que le constituant charge (5) est engagé, au moment de la préparation de la composition POS, sous la forme d'une suspension obtenue :

- en mettant en présence la charge minérale renforçante avec l'agent de compatibilisation (AC) et avec une huile silicone comprenant une partie ou la totalité du (ou des) POS (1) et éventuellement avec de l'eau,
- cette mise en présence étant un traitement consistant à introduire l'AC en deux temps dans le milieu de préparation de la suspension :

  • d'une part, avant et/ou sensiblement simultanément à l'incorporation, dans au moins une partie de l'huile silicone mise en oeuvre et dans l'eau éventuellement présente, de la charge minérale renforçante particulaire utilisée, cette introduction d'AC (portion 1) s'opérant en une ou plusieurs fois avec une fraction d'AC correspondant à une proportion inférieure ou égale à 8 % en poids par rapport à la charge renforçante particulaire ; et
  • d'autre part (portion 2), après cette incorporation de la charge renforçante dans au moins une partie de l'huile silicone et dans l'eau éventuellement présente.

Pour la préparation de la suspension de la charge minérale renforçante particulaire (5) traitée à l'aide d'un AC, dans une huile silicone, on procède comme décrit dans le document WO-A-98158997 auquel l'homme de métier pourra se reporter pour avoir plus de détails.

La charge renforçante particufaire habituellement utilisée consiste dans une charge siliceuse. A titre de charges siliceuses susceptibles d'être mises en oeuvre, conviennent toutes les silices précipitées ou pyrogénées (ou silices de combustion) connues de l'homme de l'art. Bien entendu, on peut utiliser aussi des coupages de différentes silices.

On préfère les silices de précipitation et/ou les silices de combustion ayant une surface spécifique BET supérieure à 40 m$^2$/g et, plus précisément comprise entre 50 et 300 m$^2$/g. A titre plus préférentiel, on utilise les silices de combustion ayant les caractéristiques de surface spécifique mentionnées ci-avant. A titre encore plus préférentiel, on utilise les silices de combustion ayant une surface spécifique BET comprise entre 170 et 230 m$^2$/g. Généralement, cette charge renforçante présente une dimension moyenne des particules inférieures à 0,1 $\mu$m.

L'agent de compatibilisation de la portion 1 est choisi parmi des molécules qui satisfont à au moins deux critères :

- présenter une interaction forte avec la charge minérale renforçante au niveau de ses liaisons hydrogène avec elle-même, et avec l'huile silicone environnante,
- être elle-même, ou ses produits de dégradation, aisément évacués du mélange final par chauffage sous vide ou sous courant gazeux, et les composés de bas poids moléculaire sont donc préférés.

L'agent de la portion 1 pourra être par exemple :

- une organosilazane et/ou un cycloorganosilazane ; il peut s'agir de composés tels que l'hexaméthyldisilazane, le divinyl-1,3 tétraméthyl-1,1,3,3 disilazane, l'hexaméthylcyclotrisilazane, l'octaméthylcyclotétrasilazane et des mélanges de ces composés ; on préfère l'hexaméthyldisilazane (HMDZ) associé ou non au divinyl-1,3 tétraméthyl-1,1,3,3 disilazane ;
- un siloxane hydroxylé di- ou de préférence mono-fonctionnel ;
- une amine telle que l'ammoniaque ou une alkylamine de bas poids moléculaire comme la diéthylamine ;
- un acide organique de bas poids moléculaire comme les acides formique ou acétique.

Les agents de compatibilisation de la portion 2 peuvent être choisis parmi les différents silazanes rencontrés ci-dessus, pris seuls ou en mélanges entre eux ; l'hexaméthyldisilazane associé ou non au divinyltétraméthyldisilazane est préféré.

Comme il est indiqué dans le document WO-A-98/58997, la préparation de la suspension peut consister :

- à mélanger :

  - (100-v) parties en poids de l'huile silicone comprenant au moins une portion du (ou des) POS (1),
  - 0 à 5 parties en poids d'eau,
  - 20 à 80 parties en poids de charge minérale renforçante particulaire,
  - et la portion 1 d'AC représentant au plus 8 % du poids de la charge renforçante,

- à introduire au mélange la portion 2 d'AC,
- à laisser réagir, de préférence sous agitation,
- à chauffer le mélange obtenu, en choisissant un couple pression/température tel qu'il se produit une dévolatilisation d'au moins une partie de l'eau éventuellement présente et des éléments volatils,
- à refroidir si nécessaire le mélange dévolatilisé,
- et à compléter éventuellement la suspension avec le reste d'huile silicone (v parties en poids ; le symbole v allant de zéro à 50 parties en poids).

L'opération de mélange s'effectue à température et à pression normales et de préférence sous atmosphère inerte (N$_2$). II convient d'ailleurs que dans ces conditions, l'huile silicone, l'eau éventuellement présente, mais également l'agent de compatibilisation, se trouvent sous forme liquide pour faciliter le mélange.

La charge minérale renforçante représente de 10 à 50 % en poids de la suspension obtenue. En pratique, cette charge est de l'ordre de 30 $\pm$ 10 %.

Avantageusement, la proportion d'agent de compatibilisation AC introduite dans un premier temps est au plus égale à 8 % en poids de la charge minérale renforçante, et de préférence comprise entre 1 et 3 % du poids de la charge renforçante. Par ailleurs, on peut indiquer que la quantité totale d'AC est habituellement comprise entre 5 et 30 % du poids de la charge minérale renforçante, de préférence entre 10 et 20 %. Les proportions d'agent de compatibilisation AC introduites d'une part avant et/ou sensiblement simultanément à l'incorporation du mélange huile/charge (portion 1) et d'autre part après ladite incorporation (portion 2) sont respectivement 5-25 % (portion 1) et 95-75 % (portion 2) en poids dans le mélange des portions 1 et 2 de l'AC.

Avantageusement encore, la charge minérale renforçante (5) traitée par un AC est présente dans le matériau silicone selon l'invention à raison de 5 à 30 % et, de préférence, de 10 à 25 % par rapport à la masse totale du matériau silicone [ensemble I + II].

Suivant un mode préféré de mise en pratique de la préparation de la suspension, celui-ci comprend les étapes suivantes :

-1- on procède à l'homogénéisation d'un mélange comprenant tout ou partie de l'huile silicone, l'eau et la première fraction d'AC,

-2- on ajoute progressivement la charge minérale renforçante particulaire au mélange obtenu en 1,

-3- on poursuit le mélange sans chauffer,

-4- on incorpore progressivement au mélange obtenu en 3, la deuxième fraction d'AC,

-5- on poursuit le mélange sans chauffer,

-6- on dévolatilise, de préférence par chauffage à une température ≥ 100°C et de préférence sous pression réduite ou sous balayage de gaz inerte comme par exemple l'azote,

-7- on laisse éventuellement refroidir le mélange dévolatilisé,

-8- et on complète éventuellement la suspension avec le reste d'huile silicone.

Outre l'utilisation d'une charge minérale renforçante (traitée par un AC) sous forme de la suspension particulière décrite ci-avant, la présente invention repose également sur l'emploi d'un ou plusieurs agents(s) tensioactif(s) permettant de conférer un caractère hydrophile, en particulier à la surface du matériau silicone.

Dans le cadre de la présente invention, les agents tensioactifs mis en oeuvre englobent des agents tensioactifs non-ioniques, ioniques ou amphotères. Les agents utilisés seront choisis au besoin sous une forme qui les rend compatibles pour le contact avec la peau et les muqueuses, en particulier buccales : ils doivent être non toxiques, non allergisants et non irritants aux doses d'emploi.

Parmi les agents tensioactifs non ioniques, on peut citer notamment : les acides gras polyalkoxylés ; les alkylphénols polyalkoxylés ; les alcools gras polyalkoxylés ; les amides gras polyalkoxylés ou polyglycérolés ; les amines grasses polyalkoxylées ; les polymères résultant de la condensation de l'oxyde d'éthylène et/ou de l'oxyde de propylène avec l'éthylèneglycol et/ou le propylèneglycol ; les polymères résultant de la condensation de l'oxyde d'éthylène et/ou de l'oxyde de propylène avec l'éthylènediamine ; les hydrocarbures terpéniques polyalkoxylés ; les polydiorganosiloxanes comportant des motifs siloxyles porteurs d'enchaînements oxyde d'éthylène et/ou d'enchaînements oxyde de propylène ; les polydiorganosiloxanes comportant des motifs siloxyles porteurs d'enchaînements de type polyol ; les silanes ou les polysilanes polyalkoxylés ; les alkylglucosides ; les alkylpolyglucosides ; les sucroéthers ; les sucroesters ; les sucroglycérides ; les esters de sorbitan ; les composés éthoxylés de ces dérivés de sucres ; et des mélanges de ces agents tensioactifs.

Parmi les agents tensioactifs anioniques, on peut citer notamment : les alkylbenzènesulfonates, les alkylsulfates, les alkyléthersulfates, les alkylaryléthersulfates, les alkylsuccinates, les alkylcarboxylates, les dérivés alkylés d'hydrolysats de protéine, les phosphates esters d'alkyle et/ou d'alkyléther et/ou d'alkylaryléther, où le cation est en général un métal alcalin ou alcalino-terreux ; et des mélanges des agents tensioactifs précités.

Parmi les agents tensioactifs cationiques, on peut citer notamment : les halogénures de trialkylbenzylammonium ; les halogénures de tétraalkylammonium ; et des mélanges de ces agents tensioactifs.

Parmi les agents tensioactifs amphotères, on peut citer notamment : les alkylbétaïnes, les alkyldiméthylbétaïnes, les alkylamidopropylbétaïnes, les alkylamidopropyldiméthylbétaïnes, les alkyltriméthyl-sulfobétaïnes ; les dérivés d'imidazoline tels que les alkylamphoacétates, alkylamphodiacétates, alkylampho-propionates, alkylamphodipropionates ; les alkylsultaïnes, les alkylamidopropyl-hydroxysultaïnes ; les produits de condensation d'acides gras et d'hydrolysats de protéines ; les dérivés amphotères des alkylpalyamines ; les protéines et hydrolysats de protéines ; et des mélanges de ces agents tensioactifs.

Les agents tensioactifs préférés sont les agents tensioactifs non ioniques. Dans ce groupe préféré, conviennent particulièrement bien les agents tensioactifs suivants :

(a) les alcools diphatiques en $C_8$-$C_{22}$ polyalkoxylés contenant de 2 à 25 motifs alkoxylés, comme par exemple des motifs oxyéthylène (OE) et/ou oxypropylène (OP) ;

(b) les polydiorganosiloxanes comportant des motifs siloxyles porteurs d'enchaînements oxyde d'éthylène et/ou d'enchaînements oxyde de propylène ; à titre d'exemples, on peut citer les agents tensioactifs de formules I, II et III qui sont décrits dans US-A-4.657.959 dont le contenu est inclus entièrement dans la présente demande par référence ; et

(c) des mélanges d'agents tensioactifs (a) entre eux, des mélanges d'agents tensioactifs (b) entre eux et des mélanges d'un ou plusieurs agent(s) de type (a) avec un ou plusieurs agent(s) de type (b).

Le (ou les) agent(s) tensioactif(s) sont ajoutés en quantité au plus égale à 10 % et, de préférence, au plus égale à 5 % par rapport à la masse totale du matériau silicone [ensemble I + II]. Dans le cadre de l'emploi des agents tensioactifs (a), (b) et (c) qui conviennent particulièrement bien, les quantités utilisées sont plus précisément comprises :

- s'agissant du (ou des) agent(s) de type (a) : entre 0,3 et 7 % et, de préférence, entre 0,5 et 3 % par rapport à la même référence, et
- s'agissant du (ou des) agent(s) de type (b) : entre 0,05 et 3 % et, de préférence, entre 0,08 et 2 % par rapport à la même référence.

En ce qui concerne les autres constituants mis en oeuvre dans le cadre de la présente invention, on peut indiquer que pour l'huile silicone - employée dans la préparation de la suspension de charge minérale renforçante (5) traitée par un AC - on peut utiliser, outre une partie ou la totalité du (ou des) POS(1), une partie du (ou des) POS (3) non réactifs tels que définis supra.

En ce qui concerne les POS (1), il s'agit de polyorganosiloxanes qui présentent, par molécule, au moins deux groupes alcényles en $C_2$-$C_6$ liés au silicium, ces groupes étant situés dans la chaîne et/ou en bout(s) de chaîne.

Plus précisément, il s'agit de POS comprenant :

(i) des motifs siloxyles de formule :

$$T_a \, Z_b \, SiO_{\frac{4-(a+b)}{2}} \tag{1.1}$$

dans laquelle :

- T est un groupe alcényle en $C_2$-$C_6$, de préférence vinyle ou allyle,
- Z est un groupe hydrocarboné monovalent, exempt d'action défavorable sur l'activité du catalyseur et choisi, de préférence, parmi les groupes alkyles ayant de 1 à 8 atomes de carbone indus, éventuellement substitués par au moins un atome d'halogène, avantageusement, parmi les groupes méthyle, éthyle, propyle et 3,3,3-trifluoropropyle et ainsi que parmi les groupes aryles et, avantageusement, parmi les radicaux xylyle, tolyle et phényle,
- a est 1 ou 2, b est 0, 1 ou 2 et a + b est compris entre 1 et 3, de préférence entre 2 et 3,

et (2i) éventuellement des autres motifs siloxyles de formule :

$$Z_c \, SiO_{\frac{4-c}{2}} \tag{1.2}$$

dans laquelle Z a la même signification que ci-dessus et c a une valeur comprise entre 0 et 3, de préférence entre 2 et 3.

Il est avantageux que ce POS ait une viscosité comprise entre 200 et 20 000 mPa.s, et de préférence entre 500 et 5000.

Bien entendu, en cas de mélange de plusieurs huiles (1) de viscosité différentes, on prend en compte la viscosité du mélange.

Toutes les viscosités dont il est question ici correspondent à une grandeur de viscosité dynamique qui est mesurée, de manière connue en soi, à 25°C.

Le POS (1) peut être uniquement formé de motifs de formule (1.1) ou peut contenir, en outre, des motifs de formule (1.2). De même, il peut présenter une structure linéaire, ramifiée, cyclique ou en réseau.

Z est généralement choisi parmi les radicaux méthyle, éthyle et phényle, 60 % molaire (ou en nombre) au moins des radicaux Z étant des radicaux méthyle.

Des exemples de motifs siloxyles de formule (1.1) sont le motif vinyldiméthylsiloxyle, le motif vinylphénylméthylsiloxyle, le motif vinylméthylsiloxyle et le motif vinylsiloxyle.

Des exemples de motifs siloxyles de formule (1.2) sont les motifs $SiO_{4/2}$, diméthylsiloxyle, méthylphénylsiloxyle, diphénylsiloxyle, méthylsiloxyle et phénylsiloxyle.

Des exemples de POS (1) sont des composés linéaires et cycliques comme : les diméthylpolysiloxanes à extrémités diméthylvinylsilyles, les copolymères (méthylvinyl) (diméthyl)polysiloxanes à extrémités triméthylsilyles, les copolymères (méthylvinyl) (diméthyl)polysiloxanes à extrémités diméthylvinylsityles ; les méthylvinylpolysiloxanes cycliques.

En ce qui concerne les POS (2), il s'agit de polyorganosiloxanes qui présentent, par molécule, au moins deux atomes d'hydrogène liés au silicium, ces groupes Si-H étant situés dans la chaîne et/ou en bout de chaîne.

L'homme du métier sait bien que quand le POS (1) a 2 groupes alcényles par molécule, le POS (2) doit avoir de préférence au moins 3 atomes d'hydrogène par molécule. Inversement, lorsque le POS (2) a 2 atomes d'hyrogène par molécule, le POS (1) a de préférence au moins 3 groupes alcényles par molécule.

Le POS (2) est plus précisément un polyorganosiloxane comprenant :

(i) des motifs siloxyles de formule :

$$H_d \, L_e \, SiO_{\frac{4-(d+e)}{2}} \qquad (2.1)$$

dans laquelle:

- L est un groupe hydrocarboné monovalent, exempt d'action défavorable sur l'activité du catalyseur et choisi, de préférence, parmi les groupes alkyles ayant de 1 à 8 atomes de carbone inclus, éventuellement substitués par au moins un atome d'halogène, avantageusement, parmi les groupes méthyle, éthyle, propyle et 3,3,3-trifluoropropyle et ainsi que parmi les groupes aryles et, avantageusement, parmi les radicaux xylyle, tolyle et phényle,
- d est 1 ou 2, e est 0, 1 ou 2, d + e a une valeur comprise entre 1 et 3, de préférence entre 2 et 3,

et (2i) éventuellement des autres motifs siloxyles de formule moyenne :

$$L_g \, SiO_{\frac{4-g}{2}} \qquad (2.2)$$

dans laquelle L a la même signification que ci-dessus et g a une valeur comprise entre 0 et 3, de préférence entre 2 et 3.

La viscosité dynamique de ce polyorganosiloxane (2) est au moins égale à 10 mPa.s et, de préférence, elle est comprise entre 20 et 1000 mPa.s.

Le POS (2) peut être uniquement formé de motifs de formule (2.1) ou comporter en plus des motifs de formule (2.2).

Le polyorganosiloxane (2) peut présenter une structure linéaire, ramifiée, cyclique ou en réseau.

Le groupe L a la même signification que le groupe Z ci-dessus.

Des exemples de motifs de formule (2.1) sont :

$$H(CH_3)_2SiO_{1/2}, \, HCH_3SiO_{2/2}, \, H(C_6H_5)SiO_{2/2}$$

Les exemples de motifs de formule (2.2) sont les mêmes que ceux donnés plus haut pour les motifs de formule (1.2).

Des exemples de POS (2) sont des composés linéaires et cycliques comme :

- les diméthylpolysiloxanes à extrémités hydrogénodiméthylsilyle,
- les copolymères (diméthyl)(hydrogénométhyl)polysiloxanes à extrémités triméthylsilyles,
- les copolymères (diméthyl)(hydrogénométhyl)polysiloxanes à extrémités hydrogénodiméthylsilyles,
- les hydrogénométhylpolysiloxanes à extrémités triméthylsilyles,
- les hydrogénométhylpolysiloxanes cycliques.

Le rapport du nombre d'atomes d'hydrogène liés au silicium dans le POS (2) sur le nombre total de groupes à insaturation alcényle du POS (1) est compris entre 0,4 et 10, de préférence entre 1 et 5.

En ce qui concerne les POS (3) non réactifs, utilisables comme diluants, il peut s'agir avantageusement d'un polydiorganosiloxane tel qu'un polydialkylorganosiloxane à extrémités trialkylsilyles ; on préfère les polydiméthylsiloxanes à extrémités triméthylsilyles. La viscosité dynamique à 25°C des POS (3) est comprise entre 10 et 5000 mPa. s et, de préférence, entre 20 et 1000 mPa.s. Ces POS (3), quand on en utilise, sont présents à raison de 10 à 120 parties en poids et, de préférence, de 20 à 100 parties en poids pour 100 parties des POS (1) et (2).

En ce qui concerne les catalyseurs (4) des réactions de polyaddition, ils sont bien connus de l'homme de métier.

On utilise, de préférence, les composés du platine et du rhodium. On peut, en particulier, utiliser les complexes du platine et d'un produit organique décrit dans les brevets US-A-3 159 601, US-A-3 159 602, US-A-3 220 972 et les brevets européens EP-A-0 057 459, EP-A-0 188 978 et EP-A-0 190 530, les complexes du platine et d'organosiloxanes vinylés décrits dans les brevets US-A-3 419 593, US-A-3 715 334, US-A-3 377 432 et US-A-3 814 730. Le catalyseur plus spécialement préféré est à base de platine. Dans ce cas, la quantité pondérale de catalyseur (4), calculée en poids de platine-métal, est généralement comprise entre 2 et 400 ppm, de préférence entre 5 et 100 ppm basés sur le poids total des POS (1) et (2).

Suivant une disposition avantageuse de la présente invention, la composition POS I du matériau silicone peut comprendre en outre un ou plusieurs constituants complémentaires choisis dans le groupe comprenant :

(6) au moins un inhibiteur des réactions de polyaddition,
(7) une charge semi-renforçante ou de bourrage,
(8) un ou plusieurs agent(s) de coloration,
(9) un ou plusieurs biocide(s), et
(10) leurs mélanges.

Les inhibiteurs (6) sont des composés bien connus. On peut en particulier utiliser les amines organiques, les oximes organiques, des diesters de diacide carboxylique, les alcools acétyléniques, les cétones acétyléniques, les vinylméthylcyclopolysiloxanes (voir par exemple US-A-3 445 420 et US-A-3 989 667). Les alcools acétyléniques sont préférés et, dans ce contexte, l'éthynylcyclohexanol (ECH) est un inhibiteur particulièrement préféré. La concentration en inhibiteur(s), quand on en utilise, est au plus égale à 2000 ppm et, de préférence, est comprise entre 2 et 500 ppm par rapport à la masse totale des POS (1) et (2).

Pour ce qui concerne les charges (7), elles ont généralement un diamètre particulaire supérieur à 0,1 μm et elles sont choisies de préférence parmi le quartz broyé, les zircones, les argiles calcinées, les terres de diatomées, le carbonate de calcium, les silicates d'aluminium et/ou de sodium, des alumines, et des mélanges de ces espèces. Sur le plan pondéral, les charges (7), quand on en utilise, sont présentes dans te matériau silicone à raison de 5 à 50 % et, de préférence, de 10 à 30 % par rapport à la masse totale du matériau silicone [ensemble I + II].

En ce qui conceme le (ou les) agent(s) de coloration(s) (8), on peut utiliser des pigments colorés minéraux et/ou organiques.

En ce qui concerne l'agent biocide (9) qui peut être mis en oeuvre dans le matériau silicone selon l'invention, il est à noter qu'il est, de préférence, choisi dans le groupe de précurseur de chlore actif à base de composés N-chlorés comprenant :

- la chloramine B (sodium-N-chlorobenzène sulfonamide),
- la chloroamine T (sodium N-chloro-p-toluène sulfonamide),
- la dichloroamine T (N,N-dichloro-p-toluène sulfonamide),
- la N-trichlorométhylmercapto-4-cyclohexène-1,2-dicarboxylamide,
- l'halazone (acide benzoïque p-n-dichlorosulfonamide),
- la N-chlorosuccinimide,
- la trichloromélamine,
- la chloroazodine

$$\left( \begin{array}{c} H_2NCN = NCNH_2 \\ \| \quad \quad \| \\ NCl \quad \quad NCl \end{array} \right),$$

- les dérivés N-chloro des acides cyanuriques, de préférence l'acide trichloroisocyanurique et/ou le sodium dichloroisocyanurique dihydrate,
- les N-chiorohydantoïnes, de préférence la 1-bromo-3-chloro-5,5'-diméthylhydantoïne, ou la 1,3-dichloro-5,5'-diméthylhydantoïne,
- et leurs mélanges.

Ce groupe d'antiseptiques correspond sensiblement à la famille des N-chloramines qui comprend les dérivés des amines dans lesquelles une ou deux des valences de l'azote trivalent sont substituées par du chlore. En présence d'eau, les N-chloramines produisent de l'acide hypochloreux HClO ou des sels de cet acide tels que NaClO. HClO est NaClO sont des dérivés chlorés actifs, doués d'une grande capacité bactéricide, que l'on peut exploiter dans le cadre du matériau silicone selon l'invention (c'est en particulier le cas, lorsque ledit matériau est destiné à la prise d'empreintes dentaires en bouche).

Avantageusement, l'agent biocide (9) peut être associé à au moins un auxiliaire antiseptique différent des antiseptiques fonctionnant par libération de chlore et de préférence choisi dans le groupe des formulations comportant un ou plusieurs ammoniums quaternaires (par exemple, le chlorure de benzalkonium) et éventuellement au moins un activateur séquestrant, de préférence sélectionné parmi les complexants d'ions métalliques (par exemple, l'EDTA ou Acide Ethylène Diamine Tétracétique).

La concentration en agent(s) biocide(s), quand on en utilise, est au plus égale à 1 %, de préférence au plus égale à 0,8 %, et plus préférentiellement encore comprise entre 0,001 et 0,5 % en poids par rapport à la masse totale du matériau silicone [ensemble I + II].

La présente invention concerne également, dans un deuxième objet, un procédé de préparation du matériau silicone I + II tel que décrit ci-dessus. Ce procédé est caractérisé en ce qu'il consiste essentiellement à mélanger les ingrédients suivants :

(A) la suspension de charge minérale renforçante (5) traitée par un AC, telle que préparée selon le procédé tel que décrit supra et exposé en détails dans le document WO-A-98/58997, avec
(B) éventuellement un ou plusieurs POS (1) tels que définis supra,
(C) un ou plusieurs POS (2) tels que définis supra,
(D) éventuellement un ou plusieurs POS (3) tels que définis supra,
(E) un catalyseur (4) des réactions de polyaddition,
(F) éventuellement un ou plusieurs inhibiteur(s) (6) tels que définis supra,
(G) éventuellement une charge semi-renforçante ou de bourrage (7) telle que définie supra,
(H) éventuellement un ou plusieurs agent(s) de coloration (8),
(I) éventuellement un ou plusieurs agent(s) biocide(s) (9), et
(J) un ou plusieurs agent(s) tensioactif(s) II tels que définis supra.

Ce mélange s'effectue de manière traditionnelle par les moyens techniques appropriés connus de l'homme de métier.

Selon une proposition avantageuse, il est préférable que :

- la suspension (A) soit préparée en présence d'une huile silicone comprenant la totalité du (ou des) POS (1), et
- la présence des ingrédients (D) et (G) soit rendue obligatoire.

Selon une variante intéressante de ce procédé :

- on produit le matériau silicone sous forme d'un système à deux composants C, et $C_2$ destinés à être mis en contact l'un avec l'autre pour produire un élastomère réticulé par réactions de polyaddition entre les POS (1) et (2), et
- on fait en sorte que l'un seulement des composants $C_1$ et $C_2$ comprenne le catalyseur (E) et éventuellement l'un ou l'autre des POS (1) et (2).

Selon une modalité préférée de la variante du procédé décrite ci-avant, on fait en sorte que les deux composants $C_1$ et $C_2$ comprennent chacun une certaine quantité de suspension (A), les quantités considérées étant, de manière très préférentielle, sensiblement équivalentes.

La présente invention a encore pour objet l'utilisation du matériau silicone I + II, tel que décrit ci-dessus, pour la prise d'empreintes, par exemple dentaires. Cette utilisation, dans une modalité de réalisation préférée, consiste à faire en sorte que la réticulation de l'élastomère silicone s'initie par mélange des composants $C_1$ et $C_2$, à prendre l'empreinte et à laisser se poursuivre la réticulation jusqu'à ce que l'élastomère ait suffisamment réticulé et soit suffisamment dur.

Selon un autre mode d'utilisation, le matériau silicone I + II tel que décrit ci-dessus est destiné à la fabrication de tampons tels que ceux utilisés dans les techniques de tampographie, où il est intéressant de pouvoir disposer d'un matériau ayant de hautes propriétés mécaniques, dont on peut moduler l'énergie de surface par ajout d'agent(s) tensioactif(s) tout en conservant le niveau de fluidité qui est nécessaire à la fabrication des tampons par moulage. Cette autre utilisation, dans une modalité préférée de réalisation, consiste à faire en sorte que la réticulation de l'élastomère silicone s'initie par mélange des composants $C_1$ et $C_2$, à conformer par moulage, de manière connue en soi, un objet ayant la forme du tampon souhaité et à laisser se poursuivre la réticulation jusqu'à ce que l'élastomère ait suffisamment réticulé et soit suffisamment dur.

Bien que la réticulation par des réactions de polyaddition entre les POS (1) et (2) puisse être initiée et développée déjà à une température voisine de la température ambiante (23°C), on peut également réaliser la réticulation par voie thermique (en chauffant par exemple à une température allant de 60°C à 110°C) et/ou par rayonnement électromagnétique (rayonnement d'électrons accélérés ou "electron beam") et/ou par rayonnement infrarouge.

L'invention sera mieux comprise à l'aide de l'exemple qui suit et qui décrit la préparation d'un matériau silicone selon l'invention, ainsi que son évaluation en termes de fluidité, d'hydrophilie et de propriétés mécaniques.

**EXEMPLE :**

**1.** Préparation d'un matériau silicone selon l'invention se présentant sous forme d'un système à deux composants $C_1$ et $C_2$ destinés à être mis en contact l'un avec l'autre pour réaliser la prise d'empreinte.

**1.1 Préparation de la suspension de charge minérale renforçante A :**

Dans un mélangeur à bras de 100 litres, on introduit :

- 40 kg d'huile polydiméthylsiloxane bloquée à chacune des extrémités de chaîne par un motif $(CH_3)_2 ViSiO_{1/2}$, où Vi = vinyle, ayant une viscosité de 1500 mPa.s,
- 0,24 kg d'hexaméthyldisilazane, et
- 0,24 kg d'eau.

Après homogénéisation, on rajoute par portion en 100 minutes 14 kg d'une silice de combustion ayant une surface spécifique de 200 $m^2$/g. Après 60 minutes de mélange, on rajoute en 60 minutes 1,88 kg d'hexaméthyldisilazane. 120 minutes plus tard commence une phase de chauffage au cours de laquelle le mélange est placé sous courant d'azote (30 $m^3$/heure) ; le chauffage continue jusqu'à atteindre environ 140°C, température palier qui est maintenue pendant 2 heures. La suspension obtenue est alors laissée à refroidir. Ce mode opératoire est répété deux fois de suite.

**1.2 Préparation du composant $C_1$ :**

Dans un mélangeur planétaire, on introduit à 23°C les ingrédients suivants :

- 42,7 kg de la suspension,
- 21,3 kg de matière siliceuse à base de quartz broyé de diamètre particulaire moyen de 3 μm, commercialisée sous la dénomination SILBOND cristobalit 8000 TST, et
- 2,85 kg de base colorante pigmentaire.

L'ensemble est homogénéisé par agitation pendant 1 heure.

L'agitation est ensuite arrêtée et on ajoute :

- 27 kg d'huile polydiméthylsiloxane bloquée à chacune des extrémités de chaîne par un motif triméthylsilyle, ayant une viscosité de 50 mPa.s, et
- 3,75 kg d'huile poly(diméthyl)(hydrogénométhyl)siloxane bloquée à chacune des extrémités de chaîne par un motif $(CH_3)_2HsiO_{1/2}$, ayant une viscocité de 30 mPa.s et contenant environ 0,25 fonction Si-H pour 100 g d'huile.

On procède à une nouvelle homogénéisation par agitation pendant 30 minutes.

L'agitation est ensuite arrêtée et, après avoir au besoin amené la température de la masse à une valeur inférieure à 40°C, on ajoute :

- 1,8 kg d'un alcool aliphatique en $C_{10}$-$C_{12}$ polyalkoxylé contenant environ 4 motifs OE et environ 3 motifs OP, commercialisé sous la dénomination ANTAROX BO 327, et
- 0,2 kg d'un polydiméthylsiloxane comportant environ 8 motifs oxyde d'éthylène, commercialisé sous la dénomination SILWET L-77.

On procède à une nouvelle homogénéisation par agitation pendant 30 minutes.

L'agitation est ensuite arrêtée et, après avoir au besoin amené la température de la masse à une valeur inférieure à 40°C, on ajoute 0,0004 kg d'éthynylcyclohexanol et on homogénéise par agitation pendant 30 minutes.

Puis, sans arrêter l'agitation, on procède au dégazage de la masse en opérant à 23°C, sous une pression réduite de 266.10$^2$ Pa, pendant 15 minutes.

**1.3 Préparation du composant C$_2$ :**

Dans un mélangeur planétaire, on introduit à 23°C les ingrédients suivants :

- 45 kg de la suspension, et
- 23 kg de matière siliceuse à base de quartz broyé de diamètre particulaire moyen de 3 μm, commercialisée sous la dénomination SILBOND cristobalit 8000 TST,

et l'ensemble est homogénéisé par agitation pendant 30 minutes.

L'agitation est ensuite arrêtée et, après avoir au besoin amené la température de la masse à une valeur inférieure à 50°C, on ajoute :

- 32 kg d'huile polydiméthylsiloxane bloquée à chacune des extrémités de chaîne par un motif triméthylsilyle, ayant une viscosité de 50 mPa.s, et
- 0,04 kg d'une solution dans le divinyltétraméthyldisiloxane d'un complexe de platine à environ 10 % en poids de platine zéro ligandé par du divinyltétraméthyldisiloxane (catalyseur dit de Karstedt).

On procède à une nouvelle homogénéisation par agitation pendant 1 heure.

**1.4 Préparation du matériau silicone selon l'invention :**

Il est obtenu par mélange, à température ambiante (23°C), de 50 parties en poids du composant C$_1$ avec 50 parties en poids du composant C$_2$. La réticulation du système à deux composants s'effectue à température ambiante (23°C), après réalisation du mélange.

Le mélange peut être réalisé, soit à la main dans un bécher, soit à l'aide d'une machine doseuse de laboratoire, comme par exemple, dans un laboratoire dentaire, la machine commercialisée sous la dénomination SILFEX SPENDER par la société AUSTENAL DENTAL GmbH. Dans le cas de prise d'empreintes par exemple dentaires, le mélange obtenu est introduit dans un récipient en matière plastique qui contient le modèle à reproduire et on laisse se poursuivre la réticulation jusqu'à son terme ; à la fin de la réticulation (environ au bout de 30 minutes), on démoule le modèle et on obtient un négatif en silicone. L'étape suivante consiste à couler dans le négatif en silicone du plâtre réfractaire dans le but de réaliser une parfaite copie du modèle d'origine.

**2. Evaluation des propriétés du matériau silicone selon l'invention :**

On mesure les propriétés initiales suivantes à 23°C, après avoir réalisé la préparation et le mélange des composants C$_1$ et C$_2$:

- la viscosité : elle est mesurée à l'aide d'un viscosimètre BROOKFIELD selon les indications de la norme AFNOR NFT 76106 de mai 1982;
- le temps de gel: ce temps correspond à la durée pendant laquelle le mélange des composants C$_1$ et C$_2$ conserve un comportement fluide ; au-delà de ce temps, le matériau acquiert les caractéristiques d'un élastomère ;
- le temps de prise : il correspond au temps nécessaire pour que le toucher de l'empreinte devienne non collant et que cette dernière devienne manipulable.

Pour évaluer, après réticulation, les performances de l'élastomère silicone réticulé obtenu, on mesure :

- d'une part les propriétés mécaniques suivantes, après 24 heures de réticulation dans une atmosphère régulée à 23°C et à 50 % d'humidité relative :

  - dureté Shore A, notée DSA (mesures effectuées selon les indications de la norme DIN-53505),
  - résistance à la rupture, en MPa, notée R/R, et allongement à la rupture, en %, notée A/R (mesures effectuées selon les indications de la norme ASTM-D-412),
  - résistance à la déchirure, en kN/m, notée RD (mesures effectuées selon les indications de la norme ASTM-D-624 A),

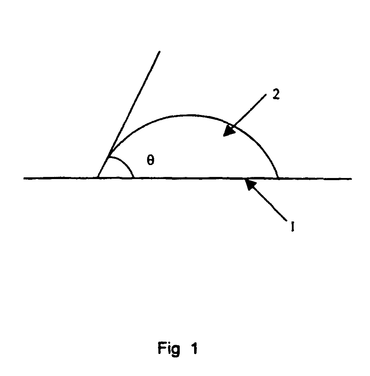

- d'autre part le caractère hydrophile, toujours après 24 heures de réticulation dans les conditions indiquées supra. La méthode consiste à déposer une microgoutte d'eau sur la surface de l'élastomère silicone réticulé et à mesurer l'angle de contact θ à l'aide d'une caméra photographique (à agrandissement d'image) et d'un goniomètre consistant dans l'appareil commercialisé sous la dénomination OLYMPUS DMS 300. Cf. la figure 1 ci-jointe en annexe où le repère 1 représente la surface du matériau silicone réticulé, le repère 2 représente la microgoutte déposée sur ladite surface et le symbole θ représente l'angle de contact de la goutte avec la surface de dépôt, qui est mesuré.

Les angles sont mesurés environ 10 secondes après le dépôt de la goutte et l'évolution de cette dernière est suivie durant une minute.

Les résultats obtenus en matière de propriétés sont rassemblés sur le tableau suivant :

| PROPRIETES | |
|---|---|
| Viscosité | 6 100 mPa.s |
| Temps de gel | 5 minutes |
| Temps de prise | 14 minutes |
| DSA après 30 minutes | 22 |
| DSA après 24 heures | 23 |
| R/R | 2,6 MPa |
| A/R | 400 % |
| RD | 6,5 kN/m |
| Angle de goutte θ | 61,1° (+/- 2,7) |

L'exemple ainsi réalisé montre que le matériau silicone selon l'invention présente, avant réticulation, une excellente fluidité marquée par une viscosité dynamique significativement inférieure à la borne de 10 000 mPa.s audessous de laquelle il est indispensable de se trouver.

Ce comportement rhéologique très favorable s'accompagne des propriétés mécaniques indispensables, c'est-à-dire : une DSA comprise dans la gamme 5-50, une R/R supérieure à 1,5 MPa, un A/R supérieur à 200 % et une RD supérieure à 5 kN/m.

La valeur d'angle de goutte habituellement observée pour les réseaux silicones hydrophobes ne comprenant pas d'agent tensioactif est de l'ordre de 100 à 105° et la goutte, en général, n'évolue pas après son dépôt. Le matériau silicone selon l'invention, après réticulation, se distingue par un caractère hydrophile marqué, avec un angle de goutte faible (61,1°) et un étalement rapide de la goutte après son dépôt. Cette évolution peut être liée à des effets de dissolution d'espèces dans l'eau qui diminuent sa tension superficielle ou à des effets de mobilité de molécules à la surface de l'élastomère silicone réticulé.

C'est la première fois, à la connaissance de la Demanderesse, qu'un pareil ensemble de caractéristiques favorables en matières de fluidité, hydrophilie et propriétés mécaniques est obtenu.

Selon un autre objet, la présente invention concerne donc encore un matériau silicone réticulable en élastomère silicone par des réactions de polyaddition, ledit matériau étant susceptible d'être obtenu par le procédé selon la revendication 8 ou 9, caractérisé par les propriétés suivantes prises en combinaison :

- avant réticulation : une viscosité dynamique inférieure à 10 000 mPa.s, et
- après réticulation pendant 24 heures dans une atmosphère régulée à 23°C et à 50 % d'humidité relative :

    - un caractère hydrophile marqué par une valeur d'angle de goutte θ, mesurée selon les indications du test décrit supra, inférieure à 90° et de préférence inférieure à 80°,
    - et un ensemble de propriétés mécaniques où la DSA se situe dans la gamme 5-50, la R/R est supérieure à 1,5 MPa.s, le A/R est supérieur à 200 % et la RD est supérieure à 5 kN/m.

Ce matériau silicone est utilisable notamment pour la prise d'empreintes, par exemple dentaires, et pour la fabrication de tampons tels que ceux utilisés dans les techniques de la tampographie.

**Revendications**

1. Matériau silicone utilisable notamment pour la prise d'empreinte qui comprend les constituants suivants :

   **I.** une composition POS réticulable par des réactions de polyaddition comprenant :

   (1) au moins un polyorganosiloxane (POS) porteur de fonctions Si-alcényles aptes à réagir par des réactions d'addition avec les fonctions réticulantes Si-H d'un POS (2),
   (2) au moins un POS porteur de fonctions Si-H aptes à réagir avec les fonctions Si-alcényles du POS (1),
   (3) éventuellement au moins un POS non réactif, différent des POS(1) et (2), utilisable comme diluant,
   (4) un catalyseur des réactions de polyaddition,
   (5) une charge minérale renforçante particulaire traitée par un agent de compatibilisation (AC);

   ladite composition ayant une viscosité dynamique inférieure à 10 000 mPa.S mesurée à l'aide d'un viscosimètre BROOKFIELD selon les indications de la norme AFNOR NFT 76106 de mai 1982;
   **II.** un agent mouillant consistant dans un ou plusieurs agents(s) tensioactifs permettant de conférer un caractère hydrophile à la surface du matériau silicone ;

   ledit matériau silicone étant **caractérisé en ce que** le constituant charge (5) est engagé, au moment de la préparation de la composition POS, sous la forme d'une suspension obtenue :

   - en mettant en présence la charge minérale renforçante avec l'agent de compatibilisation (AC) et avec une huile silicone comprenant une partie ou la totalité du (ou des) POS (1 ) et éventuellement avec de Peau,
   - cette mise en présence étant un traitement consistant à introduire l'AC en deux temps dans le milieu de préparation de la suspension :

     • d'une part, avant et/ou sensiblement simultanément à l'incorporation, dans au moins une partie de l'huile silicone mise en oeuvre et dans l'eau éventuellement présente, de la charge minérale renforçante particulaire utilisée, cette introduction d'AC (portion 1) s'opérant en une ou plusieurs fois avec une fraction d'AC correspondant à une proportion inférieure ou égale à 8 % en poids par rapport à la charge renforçante particulaire ; et
     • d'autre part (portion 2), après cette incorporation de la charge renforçante dans au moins une partie de l'huile silicone et dans l'eau éventuellement présente.

2. Matériau selon la revendication 1, **caractérisé en ce que** la préparation de la suspension de charge minérale renforçante (5) traitée par un AC consiste :

   • à mélanger:

     - (100-v) parties en poids de l'huile silicone comprenant au moins une portion du (ou des) POS (1),
     - 0 à 5 parties en poids d'eau,
     - 20 à 80 parties en poids de charge minérale renforçante particulaire,
     - et la portion 1 d'AC représentant au plus 8 % du poids de la charge renforçante,

   • à introduire au mélange la portion 2 d'AC,
   • à laisser réagir, de préférence sous agitation,
   • à chauffer le mélange obtenu, en choisissant un couple pression/température tel qu'il se produit une déyotatilisation d'au moins une partie de l'eau éventuellement présente et des éléments volatils,
   • à refroidir si nécessaire le mélange dévolatilisé,
   • et à compléter éventuellement la suspension avec le reste d'huile silicone (v parties en poids ; le symbole v allant de zéro à 50 parties en poids).

3. Matériau selon la revendication 1 ou 2, **caractérisé en ce que** :

   • l'agent de compatibilisation, dans sa portion 1, est choisi dans le groupe formé par:

     - une organosilazane et/ou un cycloorganosilazane ;
     - un siloxane hydroxylé di- ou mono-fonctionnel ;

- une amine ;
- un acide choisit parmi les acides formique ou acétique.

- l'agent de compatibilisation, dans sa portion 2, est choisi parmi un organosilazane et/ou un cycloorganosilazane.

4. Matériau selon l'une quelconque des revendications 1 à 3, **caractérisé en ce que** les agents tensioactifs sont choisis dans le groupe formé par :

- les agents tensioactifs non ioniques englobant : les acides gras polyalkoxylés ; les alkylphénols polyalkoxylés ; les alcools gras polyalkoxylés ; les amides gras polyalkoxylés ou polyglycérolés ; les amines grasses polyalkoxylées ; les polymères résultant de la condensation de l'oxyde d'éthylène et/ou de l'oxyde de propylène avec l'éthylèneglycol et/ou le propylèneglycol ; les polymères résultant de la condensation de l'oxyde d'éthylène et/ou de l'oxyde de propylène avec l'éthylènediamine ; les hydrocarbures terpéniques polyalkoxylés ; les polydiorganosiloxanes comportant des motifs siloxyles porteurs d'enchaînements oxyde d'éthylène et/ou d'enchaînements oxyde de propylène ; les polydiorganosiloxanes comportant des motifs siloxyles porteurs d'enchaînements de type polyol ; les silanes ou les polysilanes polyalkoxylés les alkylglucosides ; les alkylpolyglucosides ; les sucroéthers ; les sucroesters ; les sucroglycérides ; les esters de sorbitan ; les composés éthoxylés de ces dérivés de sucres ; et des mélanges de ces agents tensioactifs ;
- les agents tensioactifs anioniques englobant : les alkylbenzènesulfonates, les alkylsulfates, les alkyléthersulfates, les alkylaryléthersulfates, les alkylsuccinates, les alkylcarboxylates, les dérivés alkylés d'hydrolysats de protéine, les phosphates esters d'alkyle et/ou d'alkyléther et/ou d'alkylaryléther, où le cation est en général un métal alcalin ou alcalino-terreux ; et des mélanges des agents tensioactifs précités ;
- les agents tensioactifs cationiques englobant : les halogénures de trialkylbenzylammonium ; les halogénures de tétraalkylammonium ; et des mélanges de ces agents tensioactifs ;
- les agents tensioactifs amphotères englobant : les alkylbétaïnes, les alkyldiméthylbétaïnes, les alkylamidopropylbétaïnes, les alkylamidopropyldiméthylbétaïnes, les alkyltriméthyl-sulfobétaïnes ; les dérivés d'imidazoline tels que les alkylamphoacétates, alkylamphodiacétates, alkylampho-propionates, alkylamphodipropionates ; les alkylsultaïnes, les alkylamidopropyl-hydroxysultaïnes ; les produits de condensation d'acides gras et d'hydrolysats de protéines ; les dérivés amphotères des alkylpolyamines ; les protéines et hydrolysats de protéines ; et des mélanges de ces agents tensioactifs.

5. Matériau selon l'une quelconque des revendications 1 à 4, **caractérisé en ce que** le POS (1) comprend :

(i) des motifs siloxyles de formule :

$$T_a Z_b SiO_{\frac{4-(a+b)}{2}} \qquad (1.1)$$

dans laquelle :

- T est un groupe alcényle en $C_2$-$C_6$,
- Z est un groupe hydrocarboné monovalent, exempt d'action défavorable sur l'activité du catalyseur et choisi parmi les groupes alkyles ayant de 1 à 8 atomes de carbone inclus, éventuellement substitués par au moins un atome d'halogène, ainsi que parmi les groupes aryles,
- a est 1 ou 2, b est 0, 1 ou 2 et a + b est compris entre 1 et 3,

et (2i) éventuellement des autres motifs siloxyles de formule :

$$Z_c SiO_{\frac{4-c}{2}} \qquad (1.2)$$

dans laquelle Z a la même signification que ci-dessus et c a une valeur comprise entre 0 et 3.

6. Matériau selon l'une quelconque des revendications 1 à 5, **caractérisé en ce que** le POS (2) comprend :

(i) des motifs siloxyles de formule :

$$H_d \ L_e \ SiO_{\frac{4-(d+e)}{2}} \hspace{4cm} (2.1)$$

dans laquelle :

- L est un groupe hydrocarboné monovalent, exempt d'action défavorable sur l'activité du catalyseur et choisi parmi les groupes alkyles ayant de 1 à 8 atomes de carbone inclus, éventuellement substitués par au moins un atome d'halogène, ainsi que parmi les groupes aryles,
- d est 1 ou 2, e est 0, 1 ou 2, d + e a une valeur comprise entre 1 et 3,

et (2i) éventuellement des autres motifs siloxyles de formule moyenne :

$$L_g \ SiO_{\frac{4-g}{2}} \hspace{4cm} (2.2)$$

dans laquelle L a la même signification que ci-dessus et g a une valeur comprise entre 0 et 3.

7. Matériau selon l'une quelconque des revendications 1 à 6, **caractérisé en ce qu'**il comprend en outre un ou plusieurs constituants complémentaires choisis dans le groupe comprenant :

(6) au moins un inhibiteur des réactions de polyaddition,
(7) une charge semi-renforçante ou de bourrage,
(8) un ou plusieurs agent(s) de coloration,
(9) un ou plusieurs biocide(s), et
(10) leurs mélanges.

8. Procédé de préparation d'un matériau silicone I + II selon l'une quelconque des revendications 1 à 7, **caractérisé en ce qu'**il consiste essentiellement à mélanger les ingrédients suivants :

(A) la suspension de charge minérale renforçante (5) traitée par un AC, avec
(B) éventuellement un ou plusieurs POS (1),
(C) un ou plusieurs POS (2),
(D) éventuellement un ou plusieurs POS (3),
(E) un catalyseur (4) des réactions de polyaddition,
(F) éventuellement un ou plusieurs inhibiteur(s) (6),
(G) éventuellement une charge semi-renforçante ou de bourrage (7),
(H) éventuellement un ou plusieurs agent(s) de coloration (8),
(I) éventuellement un ou plusieurs agent(s) biocide(s) (9), et
(J) un ou plusieurs agent(s) tensioactif(s) II.

9. Procédé selon la revendication 8, **caractérisé par** les points suivants :

- on produit le matériau silicone sous forme d'un système à deux composants $C_1$ et $C_2$ destinés à être mis en contact l'un avec l'autre pour produire un élastomère réticulé par réactions de polyaddition entre les POS (1) et (2), et
- on fait en sorte que l'un seulement des composants $C_1$ et $C_2$ comprenne le catalyseur (E) et éventuellement l'un ou l'autre des POS (1) et (2).

10. Utilisation du matériau silicone I + II selon l'une quelconque des revendications 1 à 7 pour la prise d'empreintes.

11. Utilisation selon la revendication 10, **caractérisée en ce qu'**elle consiste à faire en sorte que la réticulation de l'élastomère silicone s'initie par mélange des composants $C_1$ et $C_2$, à prendre l'empreinte et à laisser se poursuivre la réticulation jusqu'à ce que l'élastomère ait suffisamment réticulé et soit suffisamment dur.

12. Utilisation du matériau silicone I + II selon l'une quelconque des revendications 1 à 7 pour la fabrication des tampons employés dans les techniques de tampographie.

**13.** Utilisation selon la revendication 12, **caractérisé en ce qu'**elle consiste à faire en sorte que la réticulation de l'élastomère silicone s'initie par mélange des composants $C_1$ et $C_2$, à conformer par moulage, de manière connue en soi, un objet ayant la forme du tampon souhaité et à laisser se poursuivre la réticulation jusqu'à ce que l'élastomère ait suffisamment réticulé et soit suffisamment dur.

**14.** Matériau silicone réticulable en élastomère silicone par des réactions de polyaddition, ledit matériau étant susceptible d'être obtenu par le procédé selon la revendication 8 ou 9, **caractérisé par** les propriétés suivantes prises en combinaison :

- avant réticulation : une viscosité dynamique inférieure à 10 000 mPa.s, et
- après réticulation pendant 24 heures dans une atmosphère régulée à 23°C et à 50 % d'humidité relative :,

  - un caractère hydrophile marqué par une valeur d'angle de goutte θ inférieure à 90°,
  - et un ensemble de propriétés mécaniques où la DSA se situe dans la gamme 5-50, la R/R est supérieure à 1,5 MPa.s, le A/R est supérieur à 200 % et la RD est supérieure à 5 kN/m.

**15.** Utilisation du matériau selon la revendication 14 pour la prise d'empreintes.

**16.** Utilisation du matériau selon la revendication 14 pour la fabrication des tampons employés dans les techniques de tampographie.

**Claims**

**1.** Silicone material that can be used especially for taking impressions, which comprises the following constituents:

I. a POS composition crosslinkable by polyaddition reactions comprising:

(1) at least one polyorganosiloxane (POS) carrying Si-alkenyl functional groups which are capable of reacting by addition reactions with the Si-H crosslinking functional groups of a POS(2),
(2) at least one POS carrying Si-H functional groups which are capable of reacting with the Si-alkenyl functional groups of the POS(1),
(3) optionally, at least one unreactive PCS, differing from the POS(1) and from the POS(2), which unreactive POS can be used as a diluent,
(4) a catalyst for catalysing the polyaddition reactions,
(5) a particulate reinforcing mineral filler treated by a compatibilizing agent (CA);

the said composition having a dynamic viscosity of less than 10 000 mPa.S, measured using a Brookfield viscometer according to the instructions of the AFNOR NFT76106 standard of May 1982;
II. a wetting agent consisting of one or more surfactants allowing the surface of the silicone material to be given a hydrophilic character;

the said silicone material being **characterized in that** the constituent filler (5) is taken, during preparation of the POS composition, in the form of a suspension obtained:

- by contacting the reinforcing mineral filler with the compatibilizing agent (CA) and with a silicone oil comprising, partly or completely, the POS(1) or POS(1)s and optionally with water;
- this contacting being a treatment consisting in introducing the CA in two stages into the suspension preparation medium:

  - firstly, before and/or substantially at the same time as the incorporation, into at least part of the silicone oil employed and in the water optionally present, the particulate reinforcing mineral filler used, this introduction of the CA (portion 1) being carried out in one or more stages with a CA fraction corresponding to a proportion of less than or equal to 8% by weight relative to the particulate reinforcing filler; and
  - secondly (portion 2), after this incorporation of the reinforcing filler into at least part of the silicone oil and into the water optionally present.

**2.** Material according to claim 1, **characterized in that** the preparation of the suspension of reinforcing mineral filler (5), treated with a CA consists:

- in mixing:

    - (100-v) parts by weight of the silicone oil comprising at least one portion of the POS(1) or POS(1)s,
    - 0 to 5 parts by weight of water,
    - 20 to 80 parts by weight of particulate reinforcing mineral filler, and
    - portion 1 of CA representing at most 8% of the weight of the reinforcing filler;

- in introducing portion 2 of CA into the mixture;
- in letting the mixture react, preferably with stirring;
- in heating the mixture obtained, choosing a pressure/temperature pair such that the volatile elements and at least some of the water optionally present undergo devolatilization;
- if necessary, in cooling the devolatilized mixture; and
- in optionally topping up the suspension with the rest of the silicone oil (v parts by weight; the symbol v ranging from zero to 50 parts by weight).

3. Material according to claim 1 or 2, **characterized in that**:

- the compatibilizing agent, in its portion 1, is chosen from the group formed by:

    - an organosilazane and/or a cycloorganosilazane,
    - a difunctional or monofunctional hydroxylated siloxane,
    - an amine, and
    - an acid chosen from formic acid and acetic acid; and

- the compatibilizing agent in its portion 2, is chosen from an organosilazane and/or a cycloorganosilazane.

4. Material according to any one of claims 1 to 3, **characterized in that** the surfactants are chosen from the group formed by:

- nonionic surfactants including: polyalkoxylated fatty acids; polyalkoxylated alkyl phenols; polyalkoxylated fatty alcohols; polyalkoxylated or polyglycerolated fatty amides; polyalkoxylated fatty amines; polymers resulting from the condensation of ethylene oxide and/or propylene oxide with ethylene glycol and/or propylene glycol; polymers resulting from the condensation of ethylene oxide and/or propylene oxide with ethylenediamine; polyalkoxylated terpene hydrocarbons; polydiorganosiloxanes containing siloxyl units carrying ethylene oxide chain links and/or propylene oxide chain links; polydiorganosiloxanes containing siloxyl units carrying polyol-type chain links; polyalkoxylated silanes or polysilanes; alkylglucosides; alkylpolyglucosides; sucroethers; sucroesters; sucroglycerides; sorbitan esters; ethoxylated compounds of these sugar derivatives; and mixtures of these surfactants;
- anionic surfactants including: alkylbenzenesulphonates, alkyl sulphates, alkyl ether sulphates, alkylaryl ether sulphates, alkyl succinates, alkyl carboxylates, alkylated derivatives of protein hydrolysates, alkyl and/or alkyl ether and/or alkylaryl ether phosphate esters, in which the cation is in general an alkali or alkaline-earth metal; and mixtures of the aforementioned surfactants;
- cationic surfactants including: trialkylbenzylammonium halides; tetraalkylammonium halides; and mixtures of these surfactants; and
- amphoteric surfactants including: alkyl betaines, alkyldimethyl betaines, alkylamidopropyl betaines, alkylamidopropyldimethyl betaines, alkyltrimethyl sulphobetaines; imidazoline derivatives such as alkyl amphoacetates, alkyl amphodiacetates, alkyl amphopropionates, and alkyl amphodipropionates; alkyl sultaines, alkylamidopropyl hydroxysultaines; products resulting from the condensation of fatty acids and protein hydrolysates; amphoteric derivatives of alkyl polyamines; proteins and protein hydrolysates; and mixtures of these surfactants.

5. Material according to any one of claims 1 to 4, **characterized in that** the POS(1) comprises:

    (i) siloxyl units of formula:

$$T_aZ_bSiO_{\frac{4-(a+b)}{2}} \qquad\qquad (1.1)$$

in which:

- T is a $C_2$-$C_6$ alkenyl group;
- Z is a monovalent hydrocarbon group, not having any action unfavourable to the activity of the catalyst and chosen from alkyl groups having from 1 to 8 carbon atoms inclusive, optionally substituted with at least one halogen atom, and from aryl groups;
- a is 1 or 2, b is 0, 1 or 2 and a + b is between 1 and 3; and

(2i) optionally other siloxyl units of formula:

$$Z_cSiO_{\frac{4-c}{2}} \qquad\qquad (1.2)$$

in which Z has the same meaning as above and c has a value of between 0 and 3.

6. Material according to any one of claims 1 to 5, **characterized in that** the POS(2) comprises:

   (i) siloxyl units of formula:

$$H_dL_eSiO_{\frac{4-(d+e)}{2}} \qquad\qquad (2.1)$$

in which:

- L is a monovalent hydrocarbon group, having no action unfavourable to the activity of the catalyst and chosen from alkyl groups having from 1 to 8 carbon atoms inclusive, optionally substituted with at least one halogen atom, and from aryl groups;
- d is 1 or 2, e is 0, 1 or 2 and d + e has a value of between 1 and 3; and

   (2i) optionally other siloxyl units of average formula:

$$L_gSiO_{\frac{4-g}{2}} \qquad\qquad (2.2)$$

in which L has the same meaning as above and g has a value of between 0 and 3.

7. Material according to any one of claims 1 to 6, **characterized in that** it furthermore includes one or more complementary constituents chosen from the group comprising:

   (6) at least one inhibitor for the polyaddition reactions;
   (7) a semi-reinforcing or bulking filler;
   (8) one or more colouring agents;
   (9) one or more biocides; and
   (10) mixtures thereof.

8. Process for preparing a silicone material I + II according to any one of claims 1 to 7, **characterized in that** it essentially consists in mixing the following ingredients:

   (A) the suspension of reinforcing mineral filler (5) treated by a CA with
   (B) optionally, one or more POS(1)s;
   (C) one or more POS(2)s;
   (D) optionally, one or more POS(3)s;
   (E) a catalyst (4) for catalysing the polyaddition reactions;

(F) optionally, one or more inhibitors (6);
(G) optionally, a semi-reinforcing or bulking filler (7);
(H) optionally, one or more colouring agents (8) ;
(I) optionally, one or more biocidal agents (9); and
(J) one or more surfactants II.

9. Process according to claim 8, **characterized by** the following points:

- the silicone material is produced in the form of a system based on two components $C_1$ and $C_2$ intended to be brought into contact with each other in order to produce an elastomer crosslinked by polyaddition reactions between the POS(1)s and POS(2)s; and
- measures are taken to ensure that only one of the components $C_1$ and $C_2$ contains the catalyst (E) and possibly one or other of the POS(1)s and POS(2)s.

10. Use of the silicone material I + II according to any one of claims 1 to 7 for taking impressions.

11. Use according to claim 10, **characterized in that** it consists in taking measures to ensure that the crosslinking of the silicone elastomer is initiated by mixing the components $C_1$ and $C_2$ together, in taking the impression and in allowing the crosslinking to continue until the elastomer is sufficiently crosslinked and sufficiently hard.

12. Use of the silicone material I + II according to any one of claims 1 to 7 for the manufacture of pads used in pad printing techniques.

13. Use according to claim 12, **characterized in that** it consists in taking measures to ensure that the crosslinking of the silicone elastomer is initiated by mixing the components $C_1$ and $C_2$ together, in forming, by moulding, in a manner known per se, an object having the shape of the desired pad and in allowing the crosslinking to continue until the elastomer is sufficiently crosslinked and sufficiently hard.

14. Crosslinkable silicone material made of a silicone elastomer by polyaddition reactions, the said material being able to be obtained by the process according to claim 8 or 9, **characterized by** the following properties taken in combination:

- before crosslinking: a dynamic viscosity of less than 10 000 mPa.s; and
- after crosslinking for 24 hours in an atmosphere controlled to 23°C and 50% relative humidity:

  - a hydrophilic character indicated by a value of the drop angle $\theta$ less than 90°;
  - and a set of mechanical properties in which the Shore A hardness lies within the 5-50 range, the tensile strength is greater than 1.5 mPa.s, the elongation at break is greater than 200% and the tear strength is greater than 5 kN/m.

15. Use of the material according to claim 14 for taking impressions.

16. Use of the material according to claim 14 for the manufacture of pads used in pad printing techniques.

**Patentansprüche**

1. Siliconmaterial, insbesondere verwendbar für das Abformen, das die folgenden Bestandteile umfaßt:

I. eine Zusammensetzung POS, vernetzbar durch Reaktionen der Polyaddition, umfassend:

(1) mindestens ein Polyorganosiloxan (POS) als Träger von Funktionen Si-Alkenyl, die fähig sind, durch Additionsreaktionen mit den vernetzenden Funktionen Si-H eines POS (2) zu reagieren,
(2) mindestens ein POS als Träger von Funktionen Si-H, die fähig sind, mit den Funktionen Si-Alkenyl des POS (1) zu reagieren,
(3) gegebenenfalls mindestens ein nicht reaktives POS, das sich von den POS (1) und (2) unterscheidet, verwendbar als Verdünnungsmittel,
(4) einen Katalysator der Reaktionen der Polyaddition,

(5) einen teilchenförmigen verstärkenden mineralischen Füllstoff, behandelt durch ein Kompatibilisierungsmittel (AC);

wobei die genannte Zusammensetzung eine dynamische Viskosität von unter 10.000 mPa.s besitzt, gemessen mit Hilfe eines Viskosimeters BROOKFIELD gemäß den Angaben der Norm AFNOR NFT 76106 vom Mai 1982;
II. ein Netzmittel, bestehend aus einem oder mehreren oberflächenaktiven Mitteln, die ermöglichen, der Oberfläche des Siliconmaterials einen hydrophilen Charakter zu verleihen;

wobei das genannte Siliconmaterial **dadurch gekennzeichnet ist, daß** der Bestandteil Füllstoff (5) im Moment der Herstellung der Zusammensetzung POS in Form einer Suspension eingesetzt wird, die erhalten wird:

- indem man den verstärkenden mineralischen Füllstoff mit dem Kompatibilisierungsmittel (AC) und mit einem Siliconöl, das einen Teil oder die Gesamtheit des (oder der) POS (1) umfaßt, und gegebenenfalls mit Wasser zusammenbringt,
- wobei dieses Zusammenbringen eine Behandlung ist, die darin besteht, das AC zu zwei Zeitpunkten in das Milieu der Herstellung der Suspension einzutragen:

  • einerseits vor und/oder etwa gleichzeitig mit dem Eintragen des verwendeten teilchenförmigen verstärkenden mineralischen Füllstoffes in mindestens einen Teil des eingesetzten Siliconöles und in das eventuell anwesende Wasser, wobei dieses Eintragen des AC (Portion 1) auf einmal oder mehrmals mit einer Fraktion des AC erfolgt, die einem Verhältnis von unter oder gleich 8 Gew.-% entspricht, bezogen auf den teilchenförmigen mineralischen Füllstoff; und
  • andererseits (Portion 2) nach diesem Eintragen des verstärkenden Füllstoffes in mindestens einen Teil des Siliconöles und in das eventuell anwesende Wasser.

**2.** Material nach Anspruch 1, **dadurch gekennzeichnet, daß** die Herstellung der Suspension von verstärkendem mineralischen Füllstoff (5), behandelt durch ein AC, darin besteht:

• zu vermischen:

  - (100-v) Gewichtsteile von Siliconöl, umfassend mindestens eine Portion des (oder der) POS (1),
  - 0 bis 5 Gewichtsteile Wasser,
  - 20 bis 80 Gewichtsteile des teilchenförmigen verstärkenden mineralischen Füllstoffes, und
  - die Portion 1 des AC, die höchstens 8 Gew.-% des verstärkenden Füllstoffes darstellt,

• in die Mischung die Portion 2 des AC einzutragen,
• reagieren zu lassen, vorzugsweise unter Rühren,
• die erhaltene Mischung zu erhitzen, indem man ein solches Paar Druck/Temperatur auswählt, daß sich eine Verflüchtigung von mindestens einem Teil des eventuell anwesenden Wassers und der flüchtigen Elemente vollzieht,
• wenn erforderlich, die von den flüchtigen Bestandteilen befreite Mischung abzukühlen, und
• gegebenenfalls die Suspension mit dem Rest des Siliconöles zu vervollständigen (v Gewichtsteile; das Symbol v reicht von 0 bis zu 50 Gewichtsteilen).

**3.** Material nach Anspruch 1 oder 2, **dadurch gekennzeichnet, daß**:

• das Kompatibilisierungsmittel in seiner Portion 1 aus der Gruppe gewählt wird, die gebildet wird durch:

  - ein Organosilazan und/oder ein Cycloorganosilazan;
  - ein hydroxyliertes di- oder monofunktionelles Siloxan;
  - ein Amin;
  - eine Säure, ausgewählt unter Ameisensäure oder Essigsäure;

• das Kompatibilisierungsmittel in seiner Portion 2 unter einem Organosilazan und/oder einem Cycloorganosilazan ausgewählt wird.

**4.** Material nach irgendeinem der Ansprüche 1 bis 3, **dadurch gekennzeichnet, daß** die oberflächenaktiven Mittel aus der Gruppe gewählt werden, die gebildet wird durch:

- die nichtionischen oberflächenaktiven Mittel: die polyalkoxylierten Fettsäuren; die polyalkoxylierten Fettamide oder Polyglycerine; die polyalkoxylierten Fettamine; die Polymere, resultierend aus der Kondensation von Ethylenoxid und/oder Propylenoxid mit Ethylenglycol und/oder Propylenglycol; die Polymere, resultierend aus der Kondensation von Ethylenoxid und/oder Propylenoxid mit Ethylendiamin; die terpenischen polyalkoxylierten Kohlenwasserstoffe; die Polydiorganosiloxane, umfassend Struktureinheiten Siloxyl als Träger von Ethylenoxid-Verkettungen und/oder Propylenoxid-Verkettungen; die Polydiorganosiloxane, umfassend Struktureinheiten Siloxyl als Träger von Verkettungen vom Typ Polyol; die polyalkoxylierten Silane oder Polysilane; die Alkylglucoside; die Alkylpolyglucoside; die Zuckerether; die Zuckerester; die Zuckerglyceride; die Sorbitanester; die ethoxylierten Verbindungen dieser Zuckerderivate; und die Mischungen dieser oberflächenaktiven Mittel;
- die anionischen oberflächenaktiven Mittel, die umfassen: die Alkylbenzolsulfonate, die Alkylsulfate, die Alkylethersulfate, die Alkylarylethersulfate, die Alkylsuccinate, die Alkylcarboxylate, die alkylierten Derivate von Proteinhydrolysaten, die Phosphatester von Alkyl und/oder Alkylether und/oder Alkylarylether, worin das Kation im allgemeinen ein Alkalimetall oder ein Erdalkalimetall ist; und die Mischungen dieser vorstehend genannten oberflächenaktiven Mittel;
- die kationischen oberflächenaktiven Mittel, die umfassen: die Halogenide von Trialkylbenzylammonium; die Halogenide von Tetraalkylammonium; und die Mischungen dieser oberflächenaktiven Mittel;
- die amphoteren oberflächenaktiven Mittel, die umfassen: die Alkylbetaine, die Alkyldimethylbetaïne, die Alkylamidopropylbetaïne, die Alkylamidopropyldimethylbetaïne, die Alkyltrimethyl-sulfobetaine; die Imidazolin-Derivate wie die Alkylamphoacetate, Alkylamphodiacetate, Alkylamphopropionate, Alkylamphodipropionate; die Alkylsultaïne, die Alkylamidopropyl-hydroxysultaine; die Produkte der Kondensation von Fettsäuren und Proteinhydrolysaten; die amphoteren Derivate von Alkylpolyaminen; die Proteine und Proteinhydrolysate; und die Mischungen dieser oberflächenaktiven Mittel.

5. Material nach irgendeinem der Ansprüche 1 bis 4, **dadurch gekennzeichnet, daß** das POS (1) umfaßt:

(i) Struktureinheiten Siloxyl der Formel:

$$T_a Z_b SiO_{\frac{4-(a+b)}{2}} \tag{1.1}$$

in der

- T eine Gruppe Alkenyl mit 2 bis 6 Kohlenstoffatomen ist,
- Z eine monovalente Kohlenwasserstoff-Gruppe ist, frei von einer ungünstigen Auswirkung auf die Aktivität des Katalysators und ausgewählt unter den Alkylgruppen mit 1 bis einschließlich 8 Kohlenstoffatomen, gegebenenfalls substituiert durch mindestens ein Halogenatom, sowie unter den Arylgruppen,
- a ist 1 oder 2, b ist 0, 1 oder 2 und a + b beträgt zwischen 1 und 3, und

(2i) gegebenenfalls andere Struktureinheiten Siloxyl der Formel

$$Z_c SiO_{\frac{4-c}{2}} \tag{1.2}$$

in der
Z die gleiche Bedeutung wie vorstehend besitzt und c einen Wert zwischen 0 und 3 aufweist.

6. Material nach irgendeinem der Ansprüche 1 bis 5, **dadurch gekennzeichnet, daß** das POS (2) umfaßt:

(i) Struktureinheiten Siloxyl der Formel:

$$H_d L_e SiO_{\frac{4-(d+e)}{2}} \tag{2.1}$$

in der

- L eine monovalente Kohlenwasserstoff-Gruppe ist, frei von einer ungünstigen Auswirkung auf die Aktivität des Katalysators und ausgewählt unter den Alkylgruppen mit 1 bis einschließlich 8 Kohlenstoffatomen, gegebenenfalls substituiert durch mindestens ein Halogenatom, sowie unter den Arylgruppen,
- d ist 1 oder 2, e ist 0, 1 oder 2 und d + e besitzen einen Wert zwischen 1 und 3, und

(2i) gegebenenfalls andere Struktureinheiten Siloxyl der mittle-ren Formel

$$L_g \, SiO_{\frac{4-g}{2}} \qquad\qquad (2.2)$$

in der
Z die gleiche Bedeutung wie vorstehend besitzt und c einen Wert zwischen 0 und 3 aufweist.

7. Material nach irgendeinem der Ansprüche 1 bis 6, **dadurch gekennzeichnet, daß** es außerdem einen oder mehrere ergänzende Bestandteile enthält, gewählt aus der Gruppe, die umfaßt:

(6) mindestens einen Inhibitor der Reaktionen der Polyaddition,
(7) einen halbverstärkenden Füllstoff oder Füllung,
(8) einen oder mehrere Farbstoff(e),
(9) ein oder mehrere Biozid(e), und
(10) ihre Mischungen.

8. Verfahren zur Herstellung eines Siliconmaterials I + II nach irgendeinem der Ansprüche 1 bis 7, **dadurch gekennzeichnet, daß** es im wesentlichen darin besteht, die folgenden Bestandteile zu vermischen:

(A) die Suspension von verstärkendem mineralischen Füllstoff (5), behandelt durch ein AC, mit
(B) gegebenenfalls einem oder mehreren POS (1),
(C) einem oder mehreren POS (2),
(D) gegebenenfalls einem oder mehreren POS (3),
(E) einem Katalysator (4) der Polyadditions-Reaktionen,
(F) gegebenenfalls einem oder mehreren Inhibitor(en) (6),
(G) gegebenenfalls einem halbverstärkenden Füllstoff oder einer Füllung (7),
(H) gegebenenfalls einem oder mehreren Farbstoff(en) (8),
(I) gegebenenfalls einem oder mehreren Biozid(en) (9), und
(J) einem oder mehreren oberflächenaktiven Mittel(n) II.

9. Verfahren nach Anspruch 8, **gekennzeichnet durch** die folgenden Punkte:

- man stellt das Siliconmaterial in Form eines Zweikomponenten-Systems $C_1$ und $C_2$ her, das dazu vorgesehen ist, miteinander in Kontakt gebracht zu werden, um ein Elastomer zu ergeben, vernetzt **durch** Reaktionen der Polyaddition zwischen den POS (1) und (2), und
- man geht in der Weise vor, daß nur einer der Bestandteile $C_1$ und $C_2$ den Katalysator (E) und gegebenenfalls das eine oder andere POS (1) und (2) umfaßt.

10. Verwendung des Siliconmaterials I + II nach irgendeinem der Ansprüche 1 bis 7 für das Abformen.

11. Verwendung nach Anspruch 10, **dadurch gekennzeichnet, daß** sie darin besteht, in einer solchen Weise vorzugehen, daß die Vernetzung des Siliconelastomers durch Vermischen der Bestandteile $C_1$ und $C_2$ in Gang gebracht, die Abformung vorgenommen wird und man dann die Vernetzung weiter fortschreiten läßt, bis das Elastomer ausreichend vernetzt wurde und ausreichend hart geworden ist.

12. Verwendung des Siliconmaterials I + II nach irgendeinem der Ansprüche 1 bis 7 für die Herstellung von Pfropfen bzw. Stempeln, die bei den Techniken der Tampographie verwendet werden.

13. Verwendung nach Anspruch 12, **dadurch gekennzeichnet, daß** sie darin besteht, in einer solchen Weise vorzugehen, daß die Vernetzung des Siliconelastomers durch Vermischen der Bestandteile $C_1$ und $C_2$ bei der Formgebung in an sich bekannter Weise durch Formgießen eines Objektes, das die Form des gewünschten Pfropfens besitzt, in Gang gebracht wird und man dann die Vernetzung weiter fortschreiten läßt, bis das Elastomer ausreichend vernetzt wurde und ausreichend hart geworden ist.

14. Siliconmaterial, vernetzbar zu einem Siliconelastomer durch Reaktionen der Polyaddition, wobei das genannte Material geeignet ist, gemäß dem Verfahren nach Anspruch 8 oder 9 erhalten zu werden, **gekennzeichnet durch** die folgenden, in Kombination betrachteten Eigenschaften:

- vor der Vernetzung: eine dynamische Viskosität von unter 10.000 mPa.s, und
- nach der Vernetzung während 24 Stunden in einer geregelten Atmosphäre von 23 °C und 50 % relativer Feuchte:

  - einen hydrophilen Charakter, dargestellt **durch** einen Wert des Tropfenwinkels Θ von unter 90°, und
  - einer Gesamtheit von mechanischen Eigenschaften, worin sich die DSA im Bereich von 5-50 befindet, die R/R über 1,5 MPa.s, die A/R über 200 % und die RD über 5 kN/m betragen.

15. Verwendung des Materials nach Anspruch 14 für Abformungen.

16. Verwendung des Materials nach Anspruch 14 für die Herstellung von Pfropfen bzw. Stempeln, die bei den Techniken der Tampographie verwendet werden.

θ

2

I

Fig 1